# EUROPÄISCHE PATENTANMELDUNG

(11) **EP 4 417 097 A1**
(43) Veröffentlichungstag der Anmeldung: **21.08.2024**
(21) Anmeldenummer: 23157379.1
(22) Anmeldetag: 17.02.2023
(51) Int. Cl.: A47K 5/12, B05B 11/10

(54) **VORRICHTUNG ZUM ABGEBEN EINES FLUIDS, INSBESONDERE EINES REINIGUNGS-, PFLEGE- ODER DESINFEKTIONSFLUIDS FÜR HÄNDE**

(71) Anmelder: Huonker GmbH, 78052 Villingen-Schwenningen (DE)
(72) Erfinder: HUONKER, Hans-Georg, 78052 Villingen-Schwenningen (DE)
(74) Vertreter: Westphal, Mussgnug & Partner Patentanwälte mbB

(57) **Zusammenfassung**

Die Erfindung betrifft eine Vorrichtung (10) zur Abgabe eines Fluids, insbesondere eines Reinigungs-, Pflege- oder Desinfektionsfluids für Hände, mit einem Gehäuse (20), welches einen Hohlraum (22) aufweist, in welchen ein Behältnis (40) zum Vorhalten des Fluids einbringbar ist, wobei das Behältnis (40) mit einer Pump- und Dosiereinheit (50) verbunden ist, mit welcher das Fluid aus dem Behältnis (40) heraus gefördert und abgegeben werden kann, wobei die Vorrichtung (10) eine Betätigungsvorrichtung (30) zur Betätigung der Pump- und Dosiereinheit (50) aufweist, wobei die Betätigungsvorrichtung (30) lösbar mit der Pump- und Dosiereinheit (50) verbindbar oder verbunden ist, und wobei die Vorrichtung (10) wenigstens ein erstes Befestigungsmittel (26) zur lösbaren Befestigung eines ersten Adapters (60, 60') zur Fixierung des Behältnisses (40) in dem Gehäuse (20) aufweist.

## Beschreibung

Die vorliegende Erfindung betrifft eine Vorrichtung zum Abgeben eines Fluids, insbesondere eines Reinigungs-, Pflege- oder Desinfektionsfluids für Hände.

Derartige Vorrichtungen sind beispielsweise aus der EP 3 650 128 A1 und der DE 10 58 272 A1 bekannt und werden auf vielfältige Weise angewendet. Beispielsweise in Handwerksbetrieben, bei denen die Handwerker regelmäßig ihre Hände verschmutzen, dienen derartige Vorrichtungen zum Abgeben eines Reinigungsfluids, insbesondere von Seife, um die Hände reinigen zu können. Da jedoch ein häufiges Händewaschen eine Belastung für die Haut im Bereich der Hände darstellt, werden derartige Vorrichtungen auch dazu verwendet, Pflegemittel, beispielsweise in Form von Handcreme, abzugeben. Hierdurch kann Hautkrankheiten vorgebeugt werden. Die Verwendung derartiger Pflegemittel kann für bestimmte Betriebe sogar vorgeschrieben sein.

Ein anderer, sehr wichtiger Anwendungsbereich derartiger Vorrichtungen findet sich in Einrichtungen des Gesundheitssektors, insbesondere in Krankenhäusern, Seniorenheimen und Arztpraxen. Dort werden die Vorrichtungen zum Abgeben von Desinfektionsfluiden verwendet, um die Verbreitung von Krankheitserregern wie Bakterien und Viren zu verringern.

Derartige Fluide werden in der Regel in Behältnissen vorgehalten. Die Fluide können mittels einer Pump- und Dosiereinheit, die in vielen Fällen mit einer Hand betätigt werden muss, aus dem Behältnis gefördert werden. Mit der anderen Hand wird das abgegebene Fluid aufgefangen, um es anschließend mit beiden Händen in die Haut im Bereich der Hände einzumassieren. Aufgrund der Tatsache, dass die Vorrichtungen von einer Vielzahl von Personen verwendet werden, besteht jedoch durch den Kontakt mit der Vorrichtung, der nötig ist, um die Pump- und Dosiereinheit zu betätigen, die Gefahr der Weitergabe von Krankheitserregern. Daher sind Vorrichtungen bekannt, bei denen die Pump- und Dosiereinheit mittels einer kontaktlos auslösbaren Antriebseinheit angetrieben werden kann, so dass kein Kontakt mit der Vorrichtung mehr notwendig ist. Hierdurch wird die Gefahr der Weitergabe von Krankheitserregern deutlich vermindert.

Zum Nachfüllen ist es bekannt, Fluid in das Behältnis nachzufüllen oder das leere Behältnis von der Pump- und Dosiereinheit zu trennen und gegen ein gefülltes Behältnis auszutauschen. Dabei ist die Geometrie der Behältnisse in der Regel für unterschiedliche Fluide nicht genormt. Zudem existieren auch Behältnisse, die bereits eine Pump- und Dosiereinheit aufweisen und mit dieser unlösbar verbunden sind. Es existiert daher eine Vielzahl von unterschiedlichen Vorrichtungen für unterschiedliche Behältnisse.

Die Aufgabe der vorliegenden Erfindung besteht daher darin, eine Vorrichtung zum Abgeben eines Fluids, insbesondere eines Reinigungs-, Pflege- oder Desinfektionsfluids für Hände, anzugeben, welche Behältnisse unterschiedlicher Geometrie, insbesondere auch Behältnisse mit fest daran angeordneter Pump- und Dosiereinheit ebenfalls unterschiedlicher Bauart, aufnehmen kann.

Gelöst wird die Aufgabe mit einer Vorrichtung mit den Merkmalen des Anspruchs 1.

Vorteilhafte Ausgestaltungen und Weiterbildung der Erfindung sind in den abhängigen Ansprüchen angegeben.

Eine Ausführungsform der Erfindung betrifft eine Vorrichtung zur Abgabe eines Fluids, insbesondere eines Reinigungs-, Pflege- oder Desinfektionsfluids für Hände, mit einem Gehäuse, welches einen Hohlraum aufweist, in welchen ein Behältnis zum Vorhalten des Fluids einbringbar ist, wobei das Behältnis mit einer Pump- und Dosiereinheit verbunden ist, mit welcher das Fluid aus dem Behältnis heraus gefördert und abgegeben werden kann, wobei die Vorrichtung eine Betätigungsvorrichtung zur Betätigung der Pump- und Dosiereinheit aufweist, wobei die Betätigungsvorrichtung lösbar mit der Pump- und Dosiereinheit verbindbar oder verbunden ist, und wobei die Vorrichtung wenigstens ein erstes Befestigungsmittel zur lösbaren Befestigung eines ersten Adapters zur Fixierung des Behältnisses in dem Gehäuse aufweist. Die lösbare Verbindung zwischen der Betätigungsvorrichtung und der Pump- und Dosiereinheit ermöglicht das problemlose Auswechseln eines Behältnisses einschließlich der Pump- und Dosiereinheit. Mittels des ersten Adapters kann sichergestellt werden, dass das Behältnis vorgegebener Geometrie relativ zur Betätigungsvorrichtung derart positioniert fixiert gehalten werden kann, dass die Betätigungsvorrichtung zuverlässig die Pump- und Dosiereinheit betätigen kann. Ein derartiger Adapter ermöglicht es somit, die Vorrichtung zur Abgabe des Fluids derart auszugestalten, dass mittels einer einzigen Ausgestaltung der Vorrichtung Behältnisses unterschiedlicher Geometrie, insbesondere auch einschließlich einer Pump- und Dosiereinheit, in der Vorrichtung aufgenommen und das Fluid abgegeben werden kann. Für unterschiedliche Behältnisse sind damit lediglich unterschiedliche erste Adapter erforderlich, während eine einzige Ausgestaltung der Vorrichtung selbst ausreichend ist.

Die Betätigungsvorrichtung kann dabei sowohl manuell betätigbar, beispielsweise als manuell betätigbarer Schwenkhebel, oder motorisch angetrieben betätigbar ausgebildet sein.

Gemäß einer vorteilhaften Ausgestaltung der Erfindung ist das erste Befestigungsmittel in oder an einer Rückwand des Hohlraums angeordnet. Bei einer derartigen Anordnung ist das erste Befestigungsmittel bei in der Vorrichtung angeordnetem Behältnis durch das Behältnis abgedeckt und nicht zugänglich.

Vorzugsweise ist das erste Befestigungsmittel als Aufnahmeöffnung ausgebildet, in welcher vorzugsweise ein Rastvorsprung angeordnet ist. Eine Aufnahmeöffnung ermöglicht eine besonders einfache Befestigung, beispielsweise durch einfaches Einhängen. Ein Rastvorsprung kann ein versehentliches Entfernen des Adapters erschweren und eine sichere Fixierung ermöglichen.

Gemäß einer vorteilhaften Weiterbildung der Erfindung weist der erste Adapter ein erstes Befestigungselement zum Eingriff in das erste Befestigungsmittel auf, wobei das erste Befestigungselement vorzugsweise als Rasthaken ausgebildet ist. Dadurch kann eine einfache und vorzugsweise sichere Fixierung ermöglicht werden.

Eine bevorzugte Ausgestaltung der Erfindung sieht vor, dass der erste Adapter eine Aufnahme, insbesondere eine Rastaufnahme, zur Aufnahme und Fixierung eines Abschnitts des Behältnisses und/oder der Pump- und Dosiereinheit aufweist. Das Behältnis kann somit auf einfache Art und Weise und ohne großen Aufwand rastend in dem Adapter fixiert werden, so dass eine einfache Handhabung ermöglicht werden kann.

Vorteilhafterweise ist ein Lösemittel an der Rastaufnahme angeordnet. Mittels des Lösemittels kann die rastende Fixierung zwischen Adapter und Behältnis gelöst und das Behältnis auf einfache Art und Weise aus dem Adapter wieder entnommen werden.

Es besteht die Möglichkeit, dass im Falle einer an dem Behältnis angeordneten Pump- und Dosiereinheit die Betätigungsvorrichtung direkt die Pump- und Dosiereinheit beaufschlagt. Gemäß einer alternativen Ausgestaltung der Erfindung weist der erste Adapter eine Anlagefläche zur Anlage der Betätigungsvorrichtung auf, welche je nach Ausgestaltung der Pump- und Dosiereinheit die Kraftübertragung von der Betätigungsvorrichtung auf die Pump- und Dosiereinheit verbessern kann.

Gemäß einer besonders bevorzugten Ausführungsform der Erfindung weist der erste Adapter eine Rückstellvorrichtung für die Pump- und Dosiereinheit auf. Es sind sowohl Behältnisse mit daran insbesondere unlösbar angeordneter Pump- und Dosiereinheit, welche zur Bewegung eines Pumpenstempels sowohl in eine erste Richtung, insbesondere der Richtung zur Auslösung des Spendevorgangs des Fluids, als auch in eine der ersten Richtung entgegengesetzte Rückrichtung einer Krafteinwirkung bedürfen, als auch Behältnisse mit daran insbesondere unlösbar angeordneter Pump- und Dosiereinheit bekannt, welche zur Bewegung eines Pumpenstempels lediglich in die erste Richtung einer Krafteinwirkung bedürfen und für die Bewegung in die entgegengesetzte Richtung eine integrierte Rückstellvorrichtung aufweisen. Die Bewegung in die erste Richtung wird mittels der Betätigungsvorrichtung ausgelöst. Um die Vorrichtung vielseitiger einsetzbar zu gestalten, ist vorzugsweise nicht die Betätigungsvorrichtung, sondern der erste Adapter mit einer Rückstellvorrichtung für die Pump- und Dosiereinheit ausgestattet, welcher damit insbesondere für Behältnisse ohne integrierte Rückstellvorrichtung der Pump- und Dosiereinheit geeignet ist. Ein Behältnis mit daran insbesondere unlösbar angeordneter Pump- und Dosiereinheit bekannt, welche zur Bewegung eines Pumpenstempels lediglich in die erste Richtung einer Krafteinwirkung bedarf und für die Bewegung in die entgegengesetzte Richtung eine integrierte Rückstellvorrichtung aufweist, kann jedoch in derselben Vorrichtung mit einem ersten Adapter ohne Rückstellvorrichtung eingesetzt werden.

Vorteilhafterweise weist die Vorrichtung wenigstens ein zweites Befestigungsmittel zur lösbaren Befestigung eines zweiten Adapters zur Positionierung des Behältnisses in dem Gehäuse auf. Mittels einem derartigen zweiten Adapter können insbesondere unterschiedliche Längen der Behältnisse ausgeglichen werden.

Vorzugsweise ist das zweite Befestigungsmittel in oder an einer Rückwand des Hohlraums angeordnet, wodurch es bei in dem Hohlraum angeordnetem Behältnis durch das Behältnis abgedeckt und unzugänglich ist.

Gemäß einer bevorzugten Weiterbildung der Erfindung ist das zweite Befestigungsmittel als Aufnahmetasche ausgebildet. Eine Aufnahmetasche ermöglicht eine besonders einfache Befestigung, beispielsweise durch einfaches Einhängen.

Vorzugsweise weist der zweite Adapter ein zweites Befestigungselement zum Eingriff in das zweite Befestigungsmittel auf, wobei das zweite Befestigungselement vorzugsweise als Einhängehaken ausgebildet ist. Dadurch kann eine einfache Befestigung ermöglicht werden.

Eine vorteilhafte Weiterbildung der Erfindung sieht vor, dass der zweite Adapter eine Auflagefläche für das Behältnis aufweist. Eine derartige Auflagefläche ermöglicht eine zuverlässige Unterstützung des Behältnisses und kann insbesondere vermeiden, dass das volle Gewicht des Behältnisses von dem ersten Adapter gehalten werden muss. Die Auflagefläche kann dabei geneigt angeordnet sein, um eine gute Restentleerung des Behältnisses zu ermöglichen.

Vorzugsweise ist an einem Rand der Auflagefläche zumindest ein im Winkel zur Auflagefläche angeordnetes seitliches Stützelement angeordnet. Ein derartiges seitliches Stützelement trägt insbesondere bei forminstabilen Behältnissen zur verbesserten Positionierung des Behältnisses bei.

Gemäß einer bevorzugten Weiterbildung der Erfindung ist an einem Rand der Auflagefläche im Winkel zur Auflagefläche eine Rückwand angeordnet, an welcher wenigstens ein, vorzugsweise zwei Halteelemente angeordnet sind. Die Halteelemente können ein teilweises Umgreifen des vor der Rückwand angeordneten Behältnisses ermöglichen und insbesondere verhindern, dass forminstabile Behältnisse von der Rückwand weg und von der Auflagefläche nach vorne rutschen.

Die Erfindung wird anhand der in den Figuren dargestellten Ausführungsbeispiele erläutert. Es zeigen
- Fig. 1: eine teilweise geschnittene perspektivische Ansicht eines Ausführungsbeispiels einer erfindungsgemäßen Vorrichtung zur Abgabe eines Fluids mit einem Gehäuse und einem darin angeordneten Behältnis sowie einem ersten Adapter und einem zweiten Adapter,

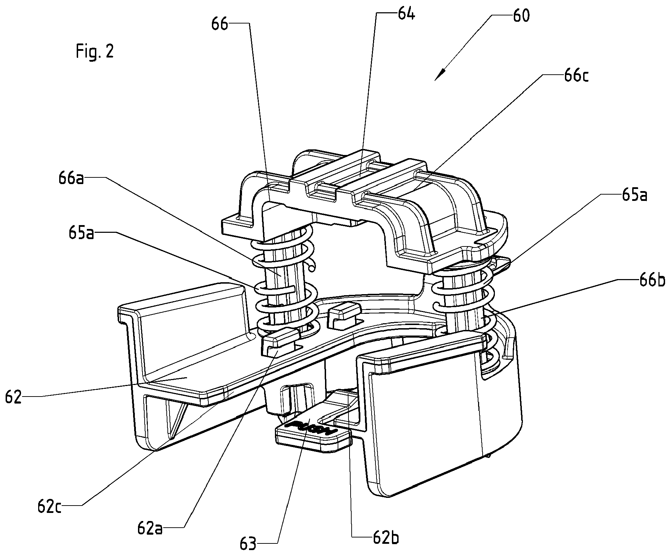

- Fig. 2: eine perspektivische Ansicht des ersten Adapters der Vorrichtung gemäß Figur 1
- Fig. 3: eine Frontansicht des ersten Adapters gemäß Figur 2,
- Fig. 4: eine Draufsicht auf den ersten Adapter gemäß Figur 2,
- Fig. 5: eine perspektivische Ansicht des ersten Adapters gemäß Figur 2 bei Anordnung in der Vorrichtung gemäß Figur 1,
- Fig. 6: einen Längsschnitt durch die Anordnung gemäß Figur 5,
- Fig. 7: eine perspektivische Ansicht einer alternativen Ausführungsform eines ersten Adapters,
- Fig. 8: eine perspektivische Ansicht des ersten Adapters gemäß Figur 7 bei Anordnung in der Vorrichtung gemäß Figur 1,
- Fig. 9: einen Längsschnitt durch die Anordnung gemäß Figur 8,
- Fig. 10: eine perspektivische Ansicht des zweiten Adapters der Vorrichtung gemäß Figur 1,
- Fig. 11: eine perspektivische Ansicht des zweiten Adapters gemäß Figur 10 bei Anordnung in dem Gehäuse der Vorrichtung gemäß Figur 1,
- Fig. 12: eine perspektivische Ansicht einer alternativen Ausführungsform eines zweiten Adapters,
- Fig. 13: eine perspektivische Ansicht des zweiten Adapters gemäß Figur 12 bei Anordnung in dem Gehäuse der Vorrichtung gemäß Figur 1,
- Fig. 14: eine perspektivische Ansicht einer weiteren alternativen Ausführungsform eines zweiten Adapters,
- Fig. 15: eine perspektivische Ansicht des zweiten Adapters gemäß Figur 14 bei Anordnung in dem Gehäuse der Vorrichtung gemäß Figur 1,
- Fig. 16: eine perspektivische Ansicht einer weiteren alternativen Ausführungsform eines zweiten Adapters,
- Fig. 17: eine perspektivische Ansicht des zweiten Adapters gemäß Figur 16 bei Anordnung in dem Gehäuse der Vorrichtung gemäß Figur 11 und
- Fig. 18: eine teilweise geschnittene perspektivische Ansicht eines weiteren Ausführungsbeispiels einer erfindungsgemäßen Vorrichtung zur Abgabe eines Fluids mit einem Gehäuse und einem darin angeordneten Behältnis sowie einem ersten Adapter und einem zweiten Adapter.

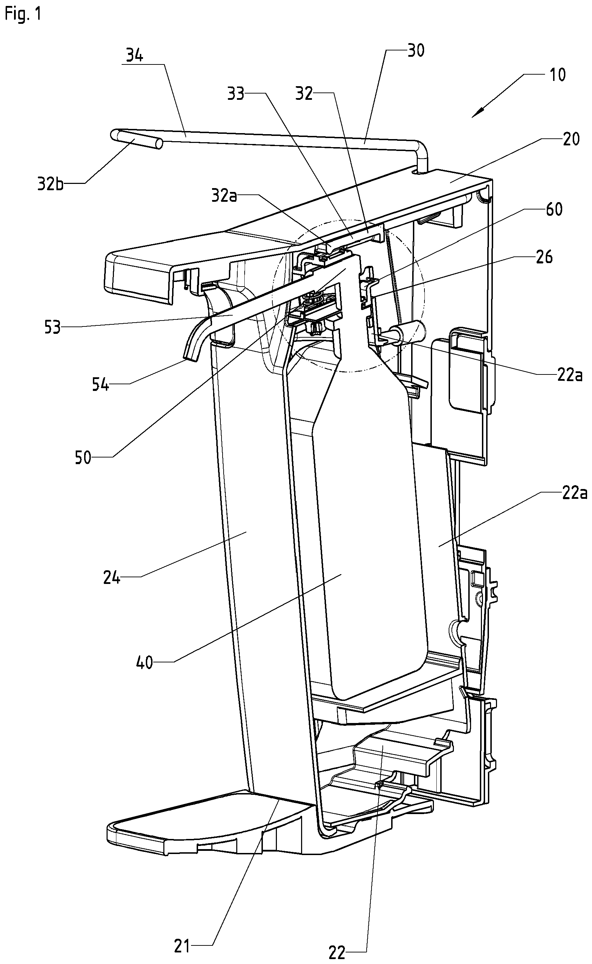

Figur 1 zeigt eine teilweise geschnittene perspektivische Ansicht eines Ausführungsbeispiels einer Vorrichtung 10 zur Abgabe eines Fluids, insbesondere eines Reinigungs-, Pflege- oder Desinfektionsfluids für Hände, mit einem Gehäuse 20, welches einen Hohlraum 22 aufweist. Das Gehäuse 20 weist insbesondere an einer Vorderseite eine Öffnung 21 auf, durch welche der Hohlraum 22 zugänglich ist. Die Öffnung 21 kann mittels einer Verschlussabdeckung 24 verschlossen werden. Das Gehäuse 20 kann aus einem undurchsichtigen Kunststoff oder Metall, beispielsweise Edelstahl, gefertigt sein. Die Verschlussabdeckung 24 kann ebenfalls aus einem undurchsichtigen Kunststoff oder Metall, beispielsweise Edelstahl, alternativ aber auch aus einem transparenten Kunststoff gefertigt sein. Die Verschlussabdeckung 24 kann an dem Gehäuse 20 schwenkbar gelagert oder auch rastend in entsprechende an dem Gehäuse 20 angeordnete Ausnehmungen einsetzbar sein.

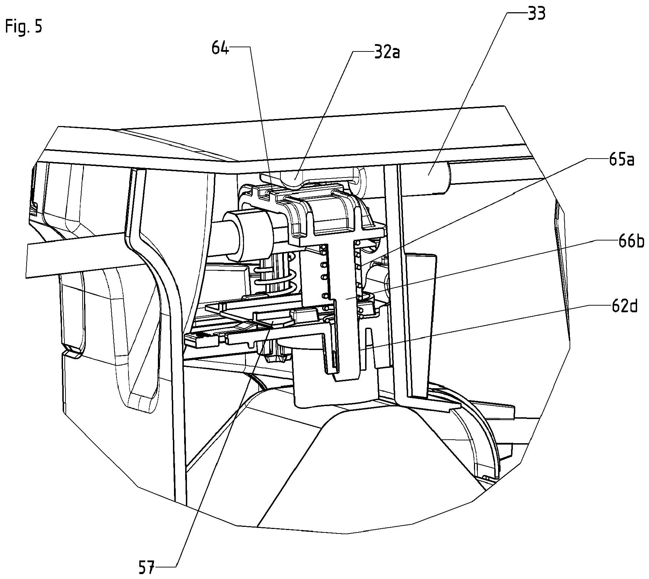

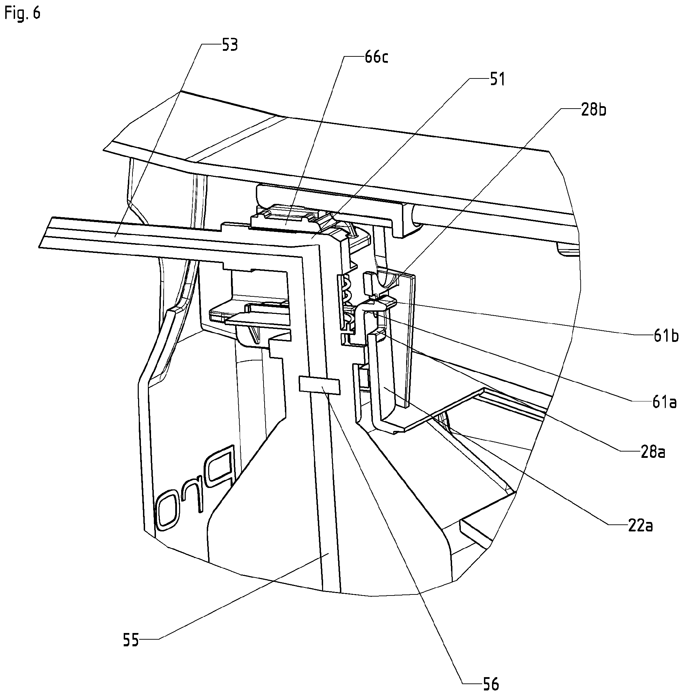

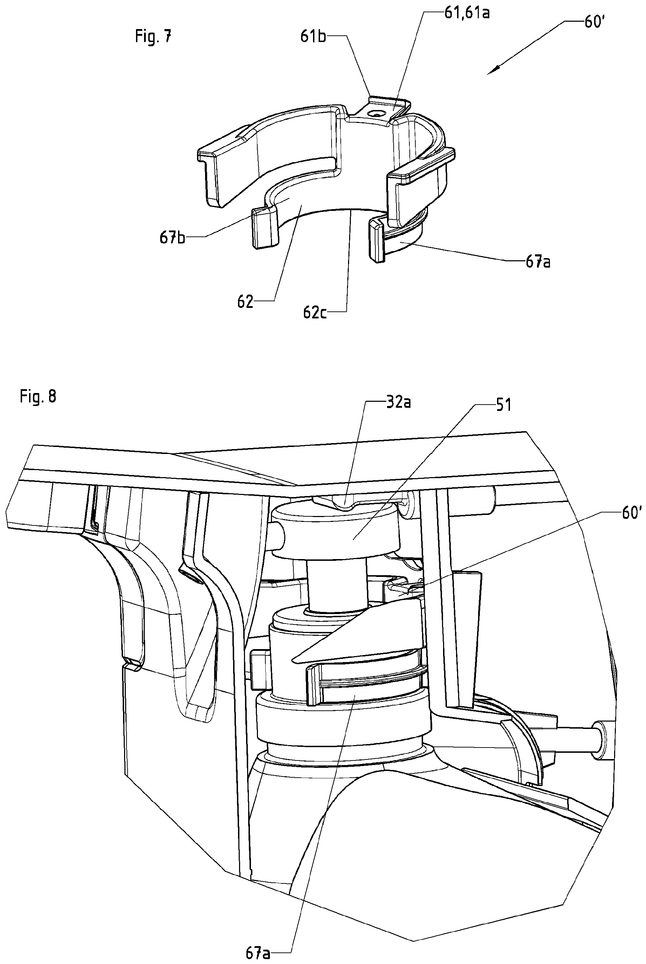

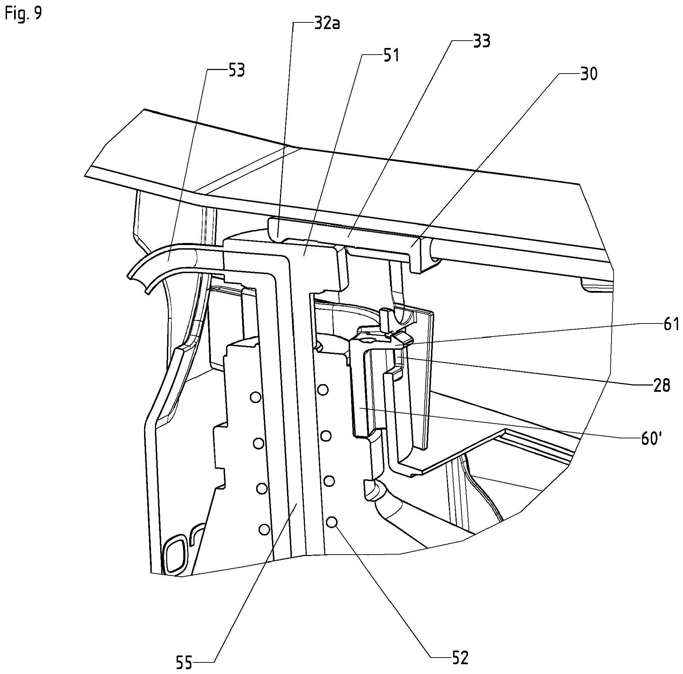

In den Hohlraum 22 ist ein Behältnis 40 zum Vorhalten des Fluids einbringbar, wobei das Behältnis 40 mit einer Pump- und Dosiereinheit 50 verbunden ist, mit welcher das Fluid aus dem Behältnis 40 heraus gefördert und abgegeben werden kann. Figur 1 zeigt das Behältnis 40 mit der Pump- und Dosiereinheit 50 lediglich abstrahiert. In Figuren 5 und 6 sind Details einer ersten Ausführungsform, in Figuren 8 und 9 Details einer weiteren Ausführungsform der Pumpt- und Dosiereinheit 50 erkennbar.

Die Pump- und Dosiereinheit 50 weist in der Regel einen Pumpenstempel 51 auf, der zum Auslösen einer Abgabe von Fluid in einer ersten Richtung x bewegbar ist. Bei dem in den Figuren 8 und 9 dargestellten Ausführungsbeispiel ist der Pumpenstempel 51 mit einem Pumpenstempel-Federelement 52 vorgespannt, während das in den Figuren 5 und 6 dargestellte Ausführungsbeispiel kein derartiges Pumpenstempel-Federelement aufweist. Der Pumpenstempel 51 umfasst einen Kanal 53, der an seinem freien Ende eine Abgabeöffnung 54 bildet, die als eine Schaumdüse ausgeführt sein kann. An seinem anderen Ende mündet der Kanal 53 in ein Saugrohr 55, welches in das Behältnis 40 hineinragt, wenn das Behältnis 40 mit der Pump- und Dosiereinheit 50 verbunden ist. An das Saugrohr 55 kann ein nicht dargestellter Verlängerungsschlauch angeschlossen werden, der insbesondere bis zum Grund des Behältnisses 40 reicht. Zwischen der AbgabeÖffnung 54 und dem Kopf des Pumpenstempels 51 kann der Kanal 53 von einem flexiblen Schlauch gebildet sein, damit Relativbewegungen zwischen dem Pumpenstempel 51 und der Abgabeöffnung 54 ausgeglichen werden können. Darüber hinaus kann die Pump- und Dosiereinheit 20 mit einem Rückschlagventil 56 versehen sein.

Wird der Pumpenstempel 51 in der ersten Richtung verschoben, insbesondere wenn, wie nachfolgend beschrieben, mittels einer Betätigungsvorrichtung 30 eine Kraft ausgeübt wird, wird das etwaig vorhandene Pumpenstempel-Federelement 52 zusammengedrückt und damit gespannt. Weiterhin verkleinert sich das Volumen zwischen der Abgabeöffnung 54 und dem Rückschlagventil 56, so dass das Fluid durch die Abgabeöffnung 54 austritt, sofern der Kanal 53 zwischen dem Rückschlagventil 56 und der Abgabeöffnung 54 vollständig mit Fluid gefüllt ist.

Übt die Betätigungsvorrichtung 30 keine Kraft mehr aus, kann der Pumpenstempel 51 entweder mittels des Pumpenstempel-Federelements 52 oder einer nachfolgend näherbeschriebenen Rückstellvorrichtung 65 entgegen der ersten Richtung x wieder zurück in die Ausgangsstellung gestellt. Aufgrund der Vergrößerung des Volumens zwischen dem Rückschlagventil 56 und der Abgabeöffnung 54 wird das Rückschlagventil 56 geöffnet und ein Teil des Fluids aus dem Behältnis 40 in das Saugrohr 55 gesaugt. Um zu verhindern, dass bereits abgegebenes Fluid oder Luft aus der Umgebung durch die Abgabeöffnung 54 zurück in den Kanal 53 gesaugt wird, kann auch in der Abgabeöffnung 54 eine Rückschlagventil-Funktion integriert sein.

Die Betätigungsvorrichtung 30 der Vorrichtung 10 ist lösbar mit der Pump- und Dosiereinheit 50 verbindbar oder verbunden. Insbesondere liegt ein erstes Ende 32a eines Betätigungshebel 32 der Betätigungsvorrichtung 30 lediglich an der Pump- und Dosiereinheit 50 direkt (vgl. Figuren 8 und 9) oder indirekt (vgl. Figuren 5 und 6) an.

Bei dem in Figur 1 dargestellten Ausführungsbeispiel der Vorrichtung 10 ist die Betätigungsvorrichtung 30 manuell betätigbar. Der Betätigungshebel 32 kann einen ersten Schenkel 33, welcher das erste Ende 32a umfasst, und einen zweiten Schenkel 34, welcher ein zweites Ende 32b umfasst, aufweisen und schwenkbar an dem Gehäuse 20 derart gelagert sein, dass der erste Schenkel 33 innerhalb des Gehäuses 20 und der zweite Schenkel 34 außerhalb des Gehäuses 20 angeordnet ist. Durch Schwenken des Betätigungshebels 32 kann Druck auf den Pumpenstempel 51 der Pump- und Dosiereinheit 50 ausgeübt werden, wenn die Pump- und Dosiereinheit 50 entsprechend relativ zu dem ersten Ende 32a des Betätigungshebels 32 positioniert ist.

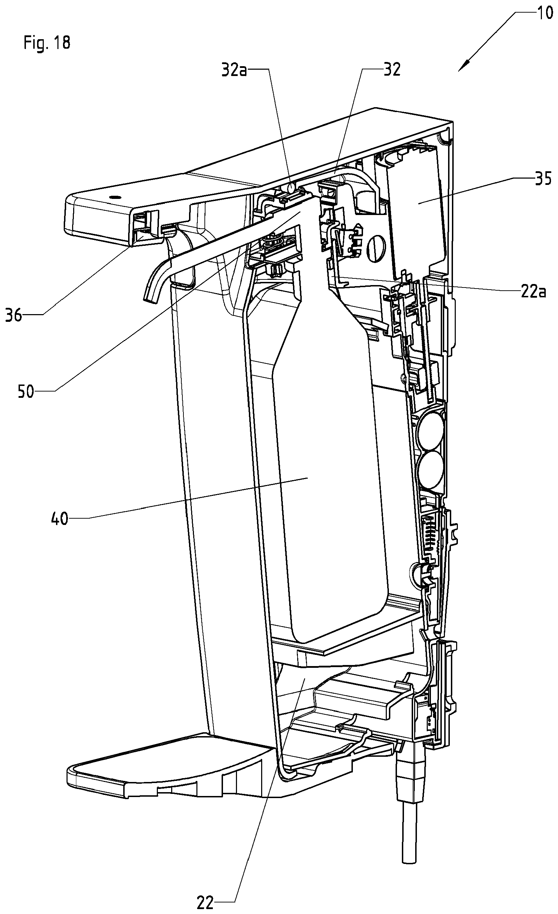

Das in Figur 18 dargestellten Ausführungsbeispiel der Vorrichtung 10 unterscheidet sich im Wesentlichen dadurch, dass die Betätigungsvorrichtung 30 motorisch angetrieben ist. Dazu ist die Vorrichtung 10 mit einer Antriebseinheit 35 ausgestattet ist, mit welcher der Betätigungshebel 32 zur Ausübung einer Kraft auf den Pumpenstempel 51 bewegt werden kann und welche beispielsweise als ein Elektromotor ausgebildet sein kann. In diesem Fall ist es nicht notwendig, dass ein Benutzer mit seiner Hand auf einen außen am Gehäuse 20 angeordneten zweiten Schenkel 34 des Betätigungshebels 32 drückt, welcher demnach in diesem Ausführungsbeispiel entfallen kann. Zum Aktivieren der Antriebseinheit 35 kann die Vorrichtung 10 des in Figur 18 dargestellten Ausführungsbeispiels einen Bewegungssensor 36 aufweisen, welcher beispielsweise als ein Ultraschallsensor oder ein Näherungssensor ausgeführt sein kann. Bei Detektion einer Annäherung wird der Betätigungshebel 32 derart bewegt, dass das erste Ende 32a den Pumpenstempel 51 in Richtung x bewegt und wie zuvor beschrieben die Abgabe des Fluids bewirkt. Insbesondere ist bei den in Figur 1 und Figur 18 beschriebenen Ausführungsbeispielen der Vorrichtung 10 das erste Ende 32a des Betätigungshebels 32 an derselben Stelle relativ zum Gehäuse 20 positioniert.

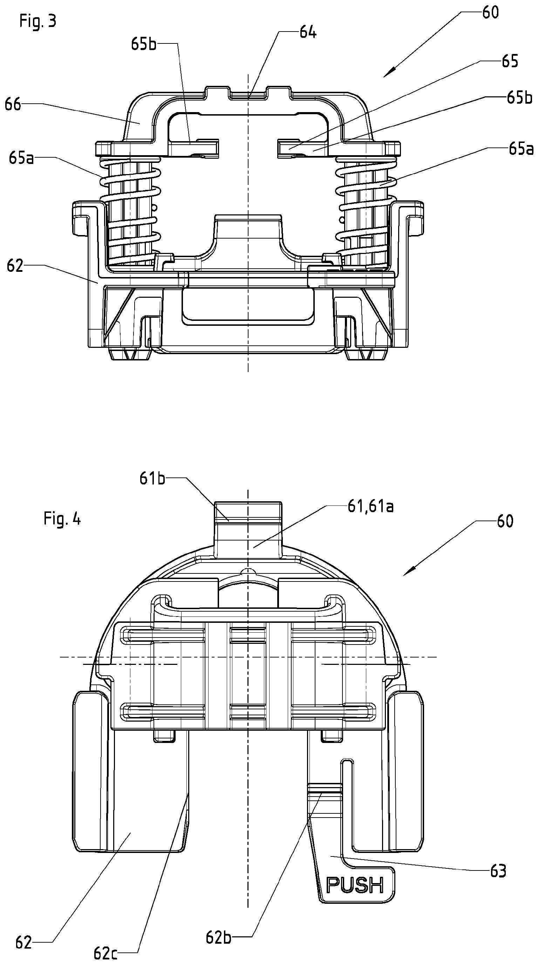

Die Vorrichtung 10 weist wenigstens ein erstes Befestigungsmittel 28 zur lösbaren Befestigung eines ersten Adapters 60 zur Fixierung des Behältnisses 40 in dem Gehäuse 20 aufweist. Ein derartiger Adapter 60 ist insbesondere derart ausgebildet, dass er die Positionierung und Fixierung des Behältnisses 40 relativ zum ersten Ende 32a des Betätigungshebels 32 sicherstellen kann, so dass Behältnisses 40 unterschiedlicher Bauart und Geometrie in die Vorrichtung 10 eingebracht werden können. Figuren 3 bis 6 zeigen Details des ersten Adapters 60.

Das erste Befestigungsmittel 28 kann in oder an einer Rückwand 22a des Hohlraums 22 angeordnet sein. Beispielsweise kann das erste Befestigungsmittel 28 als Aufnahmeöffnung 28a ausgebildet ist, in welche der Adapter 60 insbesondere mit einem ersten Befestigungselement 61 eingesetzt oder eingehängt werden kann. In der Aufnahmeöffnung 28a kann ein Rastvorsprung 28b, beispielsweise eine Rastkante, angeordnet sein. Das erste Befestigungselement 61 des ersten Adapters 60 kann beispielsweise als Rasthaken 61a ausgebildet sein und beispielsweise eine Kante 61b aufweisen, die den Rastvorsprung 28b hintergreift (vgl. Fig. 6).

Der erste Adapter 60 kann eine Rastaufnahme 62 zur Aufnahme und Fixierung eines Abschnitts des Behältnisses 40 und/oder der Pump- und Dosiereinheit 50 aufweisen. Die Pump- und Dosiereinheit 50 durchsetzt dabei insbesondere eine Ausnehmung 62c der Rastaufnahme 62. Bei dem in den Figuren 5 und 6 dargestellten Ausführungsbeispiel der Pump- und Dosiereinheit 50 ist an der Pump- und Dosiereinheit 50 ein umlaufender radialer Kragen 57 mit etwa rechteckigem Außenumfang angeordnet, welcher in eine entsprechende Führung 62a des ersten Adapters 60, insbesondere der Rastaufnahme 62, eingeschoben werden kann. Dabei kann der Kragen 57 hinter einem Rastvorsprung 62b rastend fixiert werden. Der erste Adapater 60 kann ein Lösemittel 63 an der Rastaufnahme 62 aufweisen, welcher beispielsweise als Schwenkhebel ausgebildet sein kann, mittels welchem der Rastvorsprung 62b derart verschwenkt werden kann, dass der Kragen 57 wieder aus der Führung 62b herausgezogen werden kann.

Der erste Adapter 60 kann eine Rückstellvorrichtung 65 für die Pump- und Dosiereinheit 50 aufweisen, so dass in dem ersten Adapter 60 insbesondere wie zuvor beschriebene Pump- und Dosiereinheiten 50 ohne Pumpenstempel-Federelement 52 aufgenommen werden können (vgl. insbesondere Figuren 5 und 6). Die Rückstellvorrichtung 65 kann einen U-förmigen Bügel 66 umfassen, welcher die Rastaufnahme 62 übergreift. Der Bügel 66 weist insbesondere zwei Schenkel 66a, 66b und einen Quersteg 66c auf, welcher die beiden Schenkel 66a, 66b verbindet. Die Rastaufnahme 62 weist beidseitig des Bügels 66 auf gegenüberliegenden Seiten der Rastaufnahme 62 jeweils eine Durchgangsöffnung 62d auf, welche die Schenkel 66a, 66b durchsetzen. Zwischen der Rastaufnahme 62 und dem Quersteg 66c des Bügels 66 ist wenigstens ein Federelement 65a, beispielsweise in Form von zwei um die Schenkel 66a, 66b angeordneten Schraubenfedern, angeordnet. Die freien Enden der Schenkel 66a, 66b können daher verdickt ausgebildet sein, um ein Durchrutschen durch die Durchgangsöffnungen 62d zu verhindern.

Der erste Adapter 60 kann eine Anlagefläche 64 zur Anlage der Betätigungsvorrichtung 30, insbesondere des ersten Endes 32a des Betätigungshebels 32, aufweisen, welche vorliegend beispielsweise an dem Quersteg 66c des Bügels 66 angeordnet ist. Der Kopf der Pump- und Dosiereinheit 50 wird bei Anordnung in dem ersten Adapter 60 von dem Bügel 66 übergriffen, wobei insbesondere der Quersteg 66c an dem Pumpenstempel 51 anliegen kann. Ausgehend von den Schenkeln 66a, 66b des Bügels 66 ist wenigstens ein in den Zwischenraum zwischen den beiden Schenkeln 66a, 66b ragender Vorsprung 65b, vorliegend zwei Vorsprünge 65b, angeordnet, welcher den Pumpenstempel 51 untergreift. Ist der erste Adapter 60 mittels des ersten Befestigungselements 61 in der Vorrichtung 10, insbesondere an der Rückwand 22 des Hohlraums 22, fixiert angeordnet, liegt die Betätigungsvorrichtung 30, insbesondere das erste Ende 32a des Betätigungshebels 32 vorzugsweise an dem Quersteg 66c des Bügels 66 an. Wird mittels der Betätigungsvorrichtung 30 eine Kraft ausgeübt, kann der Bügel 66 gegen die Kraft der Federelemente 65a in Richtung x verschoben werden, wodurch ebenfalls der Pumpenstempel 51 der Pump- und Dosiereinheit 50 in Richtung x verschoben wird und der Prozess zur Abgabe des Fluids initiiert wird. Wird keine Kraft mehr durch die Betätigungsvorrichtung 30 ausgeübt, kann der Pumpenstempel 51 durch die Rückstellvorrichtung 65, insbesondere die Federelemente 65a und die Vorsprünge 65b, welche den Pumpenstempel 51 untergreifen, in Richtung entgegen der ersten Richtung x wieder in die Ausgangsstellung zurückgestellt werden.

In den Figuren 7 bis 9 ist ein zweites Ausführungsbeispiel eines ersten Adapters 60' dargestellt, welche anstelle des ersten Adapters 60 in der Vorrichtung gemäß Figur 1 zur Anwendung kommen kann. Der erste Adapter 60' kann eine Rastaufnahme 62 zur Aufnahme und Fixierung eines Abschnitts des Behältnisses 40 und/oder der Pump- und Dosiereinheit 50 aufweisen. Insbesondere umfasst die Rastaufnahme 62 zwei bogenförmig gekrümmte Federarme 67a, 67b, welche einen Abschnitt des Behältnisses 40, beispielsweise einen Flaschenhals, und/oder der Pump- und Dosiereinheit 50 umgreifen, insbesondere formschlüssig an diesem anliegen, und dadurch das Behältnis 40 und/oder die Pump- und Dosiereinheit 50 positionieren und fixieren. Der erste Adapter 60' gemäß diesem Ausführungsbeispiel weist keine Rückstellvorrichtung 65 für die Pump- und Dosiereinheit 50 auf, so dass in diesem ersten Adapter 60' insbesondere wie zuvor beschriebene Pump- und Dosiereinheiten 50 mit Pumpenstempel-Federelement 52 aufgenommen werden können (vgl. Figuren 8 und 9) .

Ist der erste Adapter 60' mittels des ersten Befestigungselements 61 in der Vorrichtung 10, insbesondere an der Rückwand 22 des Hohlraums 22, fixiert angeordnet, und das Behältnis 40 mit der Pump- und Dosiereinheit 50 in dem ersten Adapter 60' fixiert, liegt die Betätigungsvorrichtung 30, insbesondere das erste Ende 32a des Betätigungshebels 32 an dem Pumpenstempel 51 an. Wird mittels der Betätigungsvorrichtung 30 eine Kraft ausgeübt, kann der Pumpenstempel 51 der Pump- und Dosiereinheit 50 in Richtung x verschoben werden und der Prozess zur Abgabe des Fluids initiiert wird. Wird keine Kraft mehr durch die Betätigungsvorrichtung 30 ausgeübt, kann der Pumpenstempel 51 durch das Pumpenstempel-Federelement 52 in Richtung entgegen der ersten Richtung x wieder in die Ausgangsstellung zurückgestellt werden.

Die Vorrichtung 10 kann wenigstens ein zweites Befestigungsmittel 28 zur lösbaren Befestigung eines zweiten Adapters 70 zur Positionierung des Behältnisses 40, insbesondere zum Ausgleich unterschiedlicher Längen des Behältnisses 40, in dem Gehäuse 20 aufweisen. Der zweite Adapter 70 ist daher derart in dem Gehäuse 20, insbesondere dem Hohlraum 22, angeordnet, dass das Behältnis 40 mit seinem Boden auf eine Auflagefläche 72 des zweiten Adapters 70 gestellt wird. Die Auflagefläche 72 kann gegen die Horizontale geneigt angeordnet sein, um eine gute Restentleerung des Behältnisses 40 ermöglichen zu können.

Das zweite Befestigungsmittel 28 kann ebenso wie das erste Befestigungsmittel 26 in oder an der Rückwand 22a des Hohlraums 22 angeordnet sein. Das zweite Befestigungsmittel 28 kann beispielsweise als Aufnahmetasche 28a ausgebildet sein.

Der zweite Adapter 70 kann ein zweites Befestigungselement 71 zum Eingriff in das zweite Befestigungsmittel 28 aufweisen, wobei das zweite Befestigungselement 71 vorzugsweise als Einhängehaken 71a ausgebildet ist.

Um zu verhindern, dass das Behältnis 40 von der Auflagefläche 72 des zweiten Adapters 70 rutscht, was insbesondere auftreten kann, wenn das Behältnis 40 forminstabil ist und beispielsweise lediglich einen Beutel umfasst, können unterschiedliche Vorkehrungen getroffen werden.

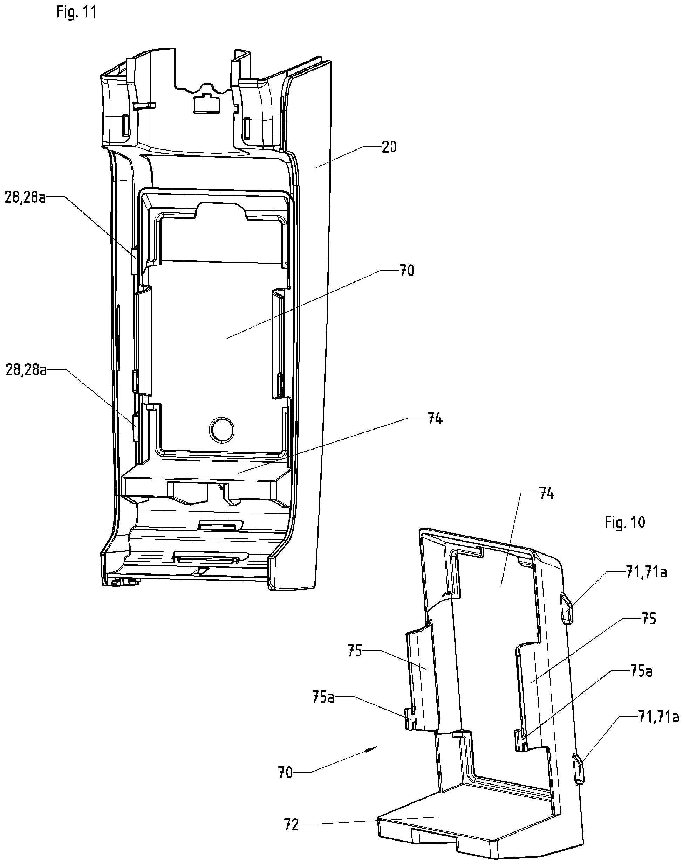

Der in Figur 10 dargestellte zweite Adapter 70 weist an einem Rand, insbesondere dem rückwärtigen Rand, der Auflagefläche 72 im Winkel zur Auflagefläche 72 eine Rückwand 74 auf, an welcher wenigstens ein, vorzugsweise zwei Halteelemente 75 angeordnet sind, welche insbesondere oberhalb der Auflagefläche 72 hervorstehen und seitlich an dem Behältnis 40, insbesondere etwa auf halber Höhe des Behältnisses 40, anliegen können. Um das Verrutschen von der geneigten Auflagefläche 72 nach vorne zu unterbinden, können an den Halteelementen 75 jeweils ein Haken 75a angeordnet sein, welche mit einem Band, beispielsweise einem Gummiband, verbunden werden können, um das Behältnis zu fixieren. Figur 11 zeigt den zweiten Adapter 70 gemäß Figur 10 im an der Rückwand 22a befestigten Zustand. Dabei ist die Rückwand 74 des zweiten Adapters 70 insbesondere im Wesentlichen parallel zur Rückwand 22a des Hohlraums 22 angeordnet.

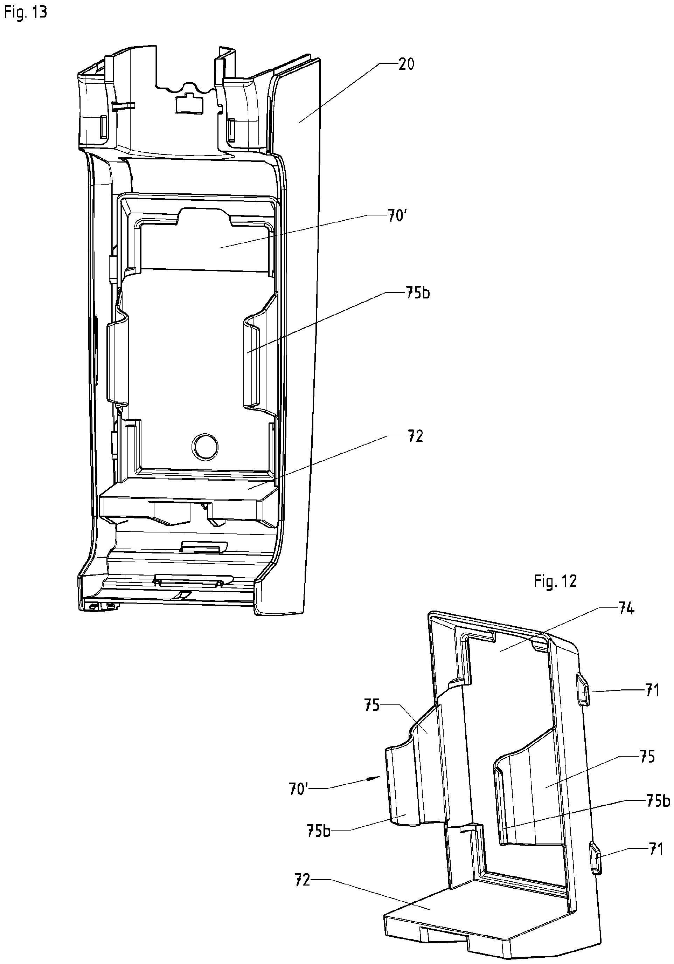

Eine alternative Ausgestaltung eines zweiten Adapters 70', welche in Figur 12 dargestellt ist, weist anstelle der Haken 75a eine Verlängerung 75b an den Halteelementen 75 auf, welche zumindest abschnittsweise aufeinander zu gekrümmt ausgebildet sind, um das Behältnis 40 zurückhalten zu können. Figur 13 zeigt den zweiten Adapter 70' gemäß Figur 12 im an der Rückwand 22a befestigten Zustand.

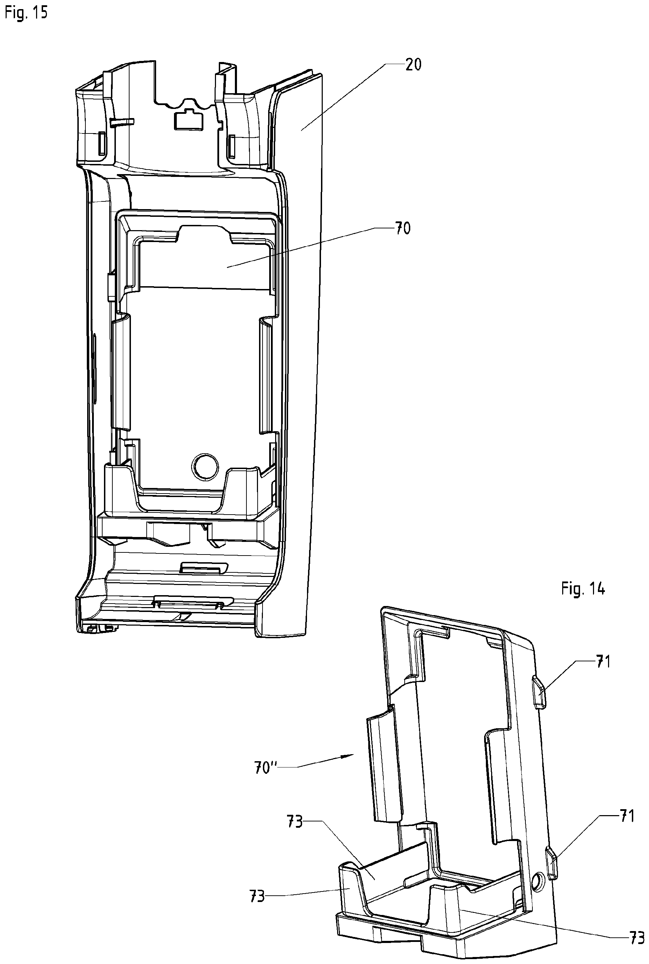

Bei einer weiteren alternative Ausgestaltung eines zweiten Adapters 70", welche in Figur 14 dargestellt ist, ist an einem Rand, insbesondere dem vorderen Rand und/oder den seitlichen Rändern, der Auflagefläche 72 zumindest ein im Winkel zur Auflagefläche 72 angeordnetes seitliches Stützelement 73 angeordnet. Dadurch wird eine Art Wanne oder Schublade gebildet, in welche der Boden des Behältnisses 40 hineingestellt und gegen Abrutschen von der Auflagefläche 72 gesichert werden kann. Figur 15 zeigt den zweiten Adapter 70" gemäß Figur 14 im an der Rückwand 22a befestigten Zustand.

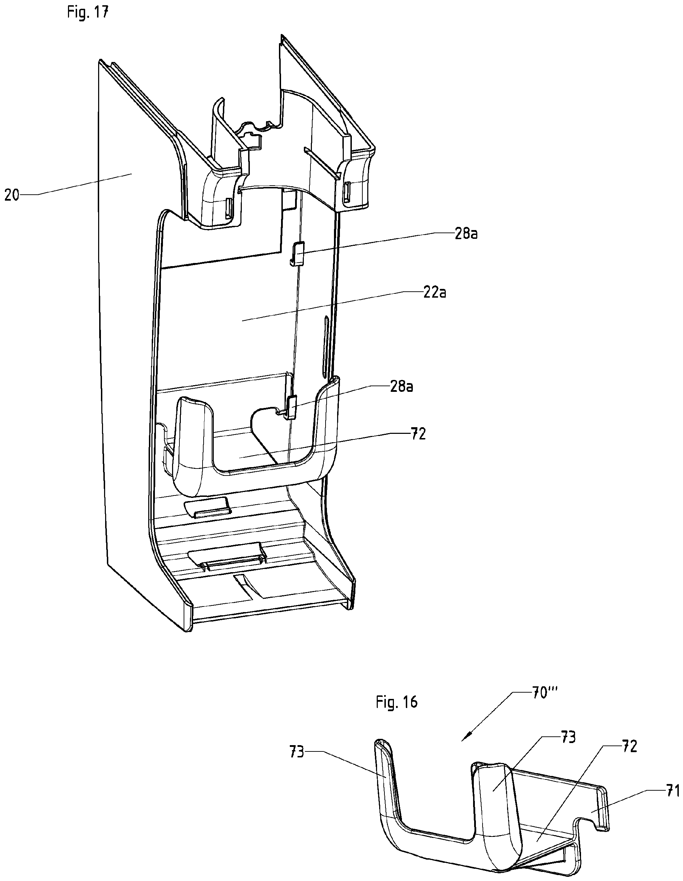

Eine weitere alternative Ausgestaltung eines zweiten Adapters 70‴, welche in Figur 16 dargestellt ist, umfasst lediglich die Auflagefläche 72 und zwei nach Art einer Gabel an der Auflagefläche 72 abgewinkelt angeordnete seitliche Stützelemente 73. Ein derartiger zweiter Adapter 70‴ kann besonders materialsparend ausgebildet werden.

### Bezugszeichenliste

- 10: Vorrichtung
- 20: Gehäuse
- 21: Öffnung
- 22: Hohlraum
- 22a: Rückwand
- 24: Verschlussabdeckung
- 26: erstes Befestigungsmittel
- 26a: Aufnahmeöffnung
- 26b: Rastvorsprung
- 28: zweites Befestigungsmittel
- 28a: Aufnahmetasche
- 30: Betätigungsvorrichtung
- 32: Betätigungshebel
- 32a: erstes Ende
- 32b: zweites Ende
- 33: erster Schenkel
- 34: zweiter Schenkel
- 35: Antriebseinheit
- 36: Bewegungssensor
- 40: Behältnis
- 50: Pump- und Dosiereinheit
- 51: Pumpenstempel
- 52: Pumpenstempel-Federelement
- 53: Kanal
- 54: Abgabeöffnung
- 55: Saugrohr
- 56: Rückschlagventil
- 57: Kragen
- 60: erster Adapter
- 61: erstes Befestigungselement
- 61a: Rasthaken
- 61b: Kante
- 62: Rastaufnahme
- 62a: Führung
- 62b: Rastvorsprung
- 62c: Ausnehmung
- 62d: Durchgangsöffnung
- 63: Lösemittel
- 64: Anlagefläche
- 65: Rückstellvorrichtung
- 65a: Federelement
- 65b: Vorsprung
- 66: Bügel
- 66a: Schenkel
- 66b: Schenkel
- 66c: Quersteg
- 67a: Federarm
- 67b: Federarm
- 60': erster Adapter
- 70: zweiter Adapter
- 71: zweites Befestigungselement
- 71a: Einhängehaken
- 72: Auflagefläche
- 73: seitliches Stützelement
- 74: Rückwand
- 75: Halteelement
- 75a: Haken
- 75b: Verlängerung
- X: erste Richtung

## Patentansprüche

1. Vorrichtung (10) zur Abgabe eines Fluids, insbesondere eines Reinigungs-, Pflege- oder Desinfektionsfluids für Hände, mit einem Gehäuse (20), welches einen Hohlraum (22) aufweist, in welchen ein Behältnis (40) zum Vorhalten des Fluids einbringbar ist, wobei das Behältnis (40) mit einer Pump- und Dosiereinheit (50) verbunden ist, mit welcher das Fluid aus dem Behältnis (40) heraus gefördert und abgegeben werden kann,
**dadurch gekennzeichnet, dass** die Vorrichtung (10) eine Betätigungsvorrichtung (30) zur Betätigung der Pump- und Dosiereinheit (50) aufweist, wobei die Betätigungsvorrichtung (30) lösbar mit der Pump- und Dosiereinheit (50) verbindbar oder verbunden ist, und wobei die Vorrichtung (10) wenigstens ein erstes Befestigungsmittel (26) zur lösbaren Befestigung eines ersten Adapters (60, 60') zur Fixierung des Behältnisses (40) in dem Gehäuse (20) aufweist.

2. Vorrichtung nach einem der vorhergehenden Ansprüche,
**dadurch gekennzeichnet, dass** das erste Befestigungsmittel (26) in oder an einer Rückwand (22a) des Hohlraums (22) angeordnet ist.

3. Vorrichtung nach einem der vorhergehenden Ansprüche,
**dadurch gekennzeichnet, dass** das erste Befestigungsmittel (26) als Aufnahmeöffnung (26a) ausgebildet ist, in welcher vorzugsweise ein Rastvorsprung (26b) angeordnet ist.

4. Vorrichtung nach einem der vorhergehenden Ansprüche,
**dadurch gekennzeichnet, dass** der erste Adapter (60, 60') ein erstes Befestigungselement (61) zum Eingriff in das erste Befestigungsmittel (26) aufweist, wobei das erste Befestigungselement (61) vorzugsweise als Rasthaken (61a) ausgebildet ist.

5. Vorrichtung nach einem der vorhergehenden Ansprüche,
**dadurch gekennzeichnet, dass** der erste Adapter (60, 60') eine Aufnahme, insbesondere eine Rastaufnahme, zur Aufnahme und Fixierung eines Abschnitts des Behältnisses (40) und/oder der Pump- und Dosiereinheit (50) aufweist.

6. Vorrichtung nach einem der vorhergehenden Ansprüche,
**dadurch gekennzeichnet, dass** ein Lösemittel (63) an der Rastaufnahme angeordnet ist.

7. Vorrichtung nach einem der vorhergehenden Ansprüche,
**dadurch gekennzeichnet, dass** der erste Adapter (60) eine Anlagefläche (64) zur Anlage der Betätigungsvorrichtung (30) aufweist.

8. Vorrichtung nach einem der vorhergehenden Ansprüche,
**dadurch gekennzeichnet, dass** der erste Adapter (60) eine Rückstellvorrichtung (65) für die Pump- und Dosiereinheit (50) aufweist.

9. Vorrichtung nach einem der vorhergehenden Ansprüche,
**dadurch gekennzeichnet, dass** die Vorrichtung (10) wenigstens ein zweites Befestigungsmittel (28) zur lösbaren Befestigung eines zweiten Adapters (70, 70', 70", 70''') zur Positionierung des Behältnisses (40) in dem Gehäuse (20) aufweist.

10. Vorrichtung nach einem der vorhergehenden Ansprüche,
**dadurch gekennzeichnet, dass** das zweite Befestigungsmittel (28) in oder an einer Rückwand (22a) des Hohlraums (22) angeordnet ist.

11. Vorrichtung nach einem der vorhergehenden Ansprüche,
**dadurch gekennzeichnet, dass** das zweite Befestigungsmittel (28) als Aufnahmetasche (28a) ausgebildet ist.

12. Vorrichtung nach einem der vorhergehenden Ansprüche,
**dadurch gekennzeichnet, dass** der zweite Adapter (70, 70', 70'', 70‴) ein zweites Befestigungselement (71) zum Eingriff in das zweite Befestigungsmittel (28) aufweist, wobei das zweite Befestigungselement (71) vorzugsweise als Einhängehaken (71a) ausgebildet ist.

13. Vorrichtung nach einem der vorhergehenden Ansprüche,
**dadurch gekennzeichnet, dass** der zweite Adapter (70, 70', 70'', 70‴) eine Auflagefläche (72) für das Behältnis (40) aufweist.

14. Vorrichtung nach einem der vorhergehenden Ansprüche,
**dadurch gekennzeichnet, dass** an einem Rand der Auflagefläche (72) zumindest ein im Winkel zur Auflagefläche (72) angeordnetes seitliches Stützelement (73) angeordnet ist.

15. Vorrichtung nach einem der vorhergehenden Ansprüche,
**dadurch gekennzeichnet, dass** an einem Rand der Auflagefläche (72) im Winkel zur Auflagefläche (72) eine Rückwand (74) angeordnet ist, an welcher wenigstens ein, vorzugsweise zwei Halteelemente (75) angeordnet sind.
